# EUROPÄISCHE PATENTSCHRIFT

(11) **EP 0 428 947 B1**
(45) Veröffentlichungstag und Bekanntmachung des Hinweises auf die Patenterteilung: **11.10.1995**
(21) Anmeldenummer: 90121478.3
(22) Anmeldetag: 09.11.1990
(51) Int. Cl.: C12P 19/26

(54) **Enzymatisches Verfahren zur Herstellung von N-Acetylneuraminsäure**
Enzymatical method for N-acetylneuramin acid production
Méthode enzymatique pour la production d'acides neuraminés N-acétylés

(30) Priorität: 15.11.1989 DE 3937891
(43) Veröffentlichungstag der Anmeldung: 29.05.1991
(73) Patentinhaber: FORSCHUNGSZENTRUM JÜLICH GMBH, 52425 Jülich (DE); CIBA-GEIGY AG, 4002 Basel (CH)
(72) Erfinder: Kragl, Udo, W-5170 Jülich (DE); Wandrey, Christian, Prof., W-5170 Jülich (DE); Ghisalba, Oreste, CH-4153 Reinach BL (CH); Gygax, Daniel, CH-4204 Himmelried (CH)

(56) Entgegenhaltungen:
- JOURNAL OF THE AMERICAN CHEMICAL SOCIETY. vol. 110, 1988, GASTON, PA US pages6481 - 6486; KIM, M.J. et al.: "Enzymes in carbohydrate synthesis: N-acetylneuraminic acid aldolase catalyzed reactions and preparation of N-acetyl-2-deoxy-D neuraminic acid derivatives"
- J. AM. CHEM. SOC. vol. 110, no. 21, 1988, pages 7159 - 7163; SIMON, E.S. et al.: "Synthesis of CMP-NeuAc from N-acetylglucosamine : generation of CTP from CMP
- using adenylate kinase"
- CHEMICAL ABSTRACTS, vol. 68, 1968 Columbus, Ohio, USA KENT, P.W. et al.:"Biosynthesis of intestinal mucins: Sialic acids of sheep colonic epithelialmucin" page 5596; column 2; ref. no. 57965B
- CHEMICAL ABSTRACTS, vol. 105, 1986 Columbus, Ohio, USA HAGEMEIER,C. et al.:"Studies on the biosynthesis of N-acetylneuraminic acid in cultured arterialwall cells" page 427; column 2; ref. no. 13147N
- Tetrahedron vol 25 no. 41 1984. pp 4663-4664

## Beschreibung

Die Erfindung bezieht sich auf ein Verfahren zur Herstellung von N-Acetylneuraminsäure durch (a) Isomerisierung von N-Acetylglucosamin in Gegenwart von N-Acylglucosamin-2-Epimerase (E.C. 5.1.3.8) zu N-Acetylmannosamin, das dann (b) in Gegenwart von N-Acetylneuraminsäure-Pyruvat-Lyase (E.C. 4.1.3.3) mit Brenztraubensäure zu N-Acetylneuraminsäure umgesetzt wird.

N-Acetylneuraminsäure (im folgenden abgekürzt als Neu5Ac) ist der wichtigste Vertreter der Stoffklasse der Sialinsäuren. Sialinsäuren besetzen die Glycid-Enden von Glycokonjugaten wie Glycolipiden und Glycoproteinen, die sich z.B. auf den Zelloberflächen befinden und wichtige Funktionen bei der Differenzierung, Reifung und intrazellulären Wechselwirkung von Zellen ausüben. Die Synthese von kurzkettigen Oligosaccchariden mit endständigen Sialinsäuren, besonders der Neu5Ac, gewinnt dabei zunehmend an Interesse. Auch über die Behandlung von Krebserkrankungen durch Neu5Ac-Derivate wird berichtet (siehe S. Sabesan u.a., J. Am. Chem. Soc. 108 (1986), 2068-2080; R. Schauer, R. Adv. Carbohydr. Chem. Biochem. 40, (1982), 131-234).

Neu5Ac wird bisher aus natürlichen Quellen isoliert (Kuhmilch, Schwalbennester; vgl. Schauer a.a.o.). Diese Quellen sind jedoch in ihrer Verfügbarkeit limitiert und die Aufreinigung ist aufgrund der Vielzahl von darin vorkommenden Sialinsäuren schwierig und zeitaufwendig.

Die enzymatische Synthese von Neu5Ac aus N-Acetylmannosamin und Brenztraubensäure (im folgenden mit ManNAc und Pyr abgekürzt) ist bereits seit den 60iger Jahren bekannt (Comb u.a., J Biol. Chem. 235 (1960), 2529-2537). Das verwendete Enzym ist die N-Acetylneuraminsäure-Pyruvat-Lyase (E.C 4.1.3.3), im folgenden kurz lyase gennant. Dabei läuft folgende Reaktion ab: 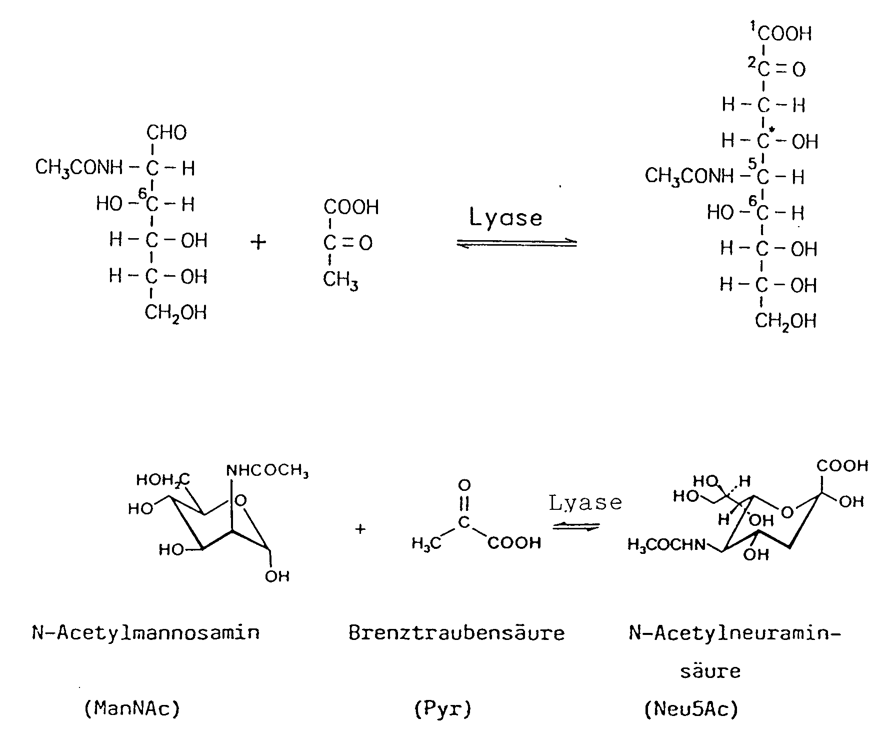
Die Isolierung und Charakterisierung der Lyase wird ausführlieh von Y. Uschida et al. im J.Biochem. 96 (1984) Seiten 507 - 522 beschrieben.

In neueren Arbeiten (M.-J. Kim u.a., J. Am. Chem. Soc. 110, (1988) 6481-6486 sowie C. Augé u.a., Tetrahedron Letters 30, (1989), 2217-2220) wird über die Synthese von Neu5Ac berichtet, bei der die Lyase in immobilisierter Form auf unlöslichen Trägern (PAN oder Agarose) kovalent angekoppelt verwendet wird. Bei dieser Form der Immobilisierung sind prinzipiell Aktivitätsverluste durch die Kopplung zu verzeichnen. Eine kontinuierliche Produktion unter Verwendung des trägerfixierten Enzymes ist nur mit zugesetzten antibakteriell wirksamen Mitteln möglich.

Ausgangsmaterial für diese N-Acetylneuraminsäurebildung ist das relativ teure N-Acetylmannosamin, auf dessen Zugänglichkeit aus N-Acetylglucosamin (GlcNAc) bereits im oben zitierten Aufsatz von M.-J. Kim hingewiesen wird, der als Möglichkeiten zur ManNAc-Gewinnung eine basenkatalysierte Isomerisierung von GLcNAc unter Bildung von ManNAc auf chemischem Wege oder die Einbeziehung einer epimerasekatalysierten Isomerisierung von GlcNAc zu ManNAc in den Herstellungsprozeß von Neu5Ac in Erwägung zieht, ohne daß jedoch irgendwelche konkreten Angaben gemacht werden, obwohl die Epimerase und deren Brauchbarkeit zur Umwandlung von GlcNAc in ManNAc seit langem bekannt waren (siehe Ghosh et al. J. of Biol. Chem. 240 (1965) 1531-6). Eine 2-Stufenreaktion durch basenkatalysierte Umsetzung von GlcNAc zu ManNAc, das extrahiert und dann mit Pyruvat-Überschuß zum NeuNAc umgewandelt wird, beschreiben E.S.Simon et al. im J.Am. Cem.Soc. 110 (1988) 7159-63.

Der vorliegenden Erfindung liegt die Aufgabe zugrunde, ein Verfahren zu schaffen, bei dem zum einen von dem billigeren GlcNAc ausgegangen wird und zum anderen irgendwelche Zwischentrennungen vermieden werden können und vorzugsweise Aktivitätsminderungen vermieden werden.

Das zu diesem Zweck entwickelte erfindungsgemäße Verfahren ist dadurch gekennzeichnet, daß man die N-Acetylneuraminsäurebildung in einem Reaktor herbeiführt, der sowohl die Epimerase als auch die Lyase enthält und in den N-Acetylglucosamin und Pyruvat eingespeist werden und aus dessen Ablauf die N-Acetylneuraminsäure gewonnen wird.

Das Verfahren nutzt in eleganter Weise die Tatsache aus, daß GlcNAc nicht von der Lyase umgesetzt wird und daß letztere relativ billig ist und daher unter Bedingungen eingesetzt werden kann, die für die Aktivität der Lyase nicht optimal sind. Es basiert auf der Erkenntnis, daß die Stabilitäts-und pH-Optima beider Enzyme ähnlich sind. Durch eine Optimierung der Reaktionsbedingungen können die Aktivitäten beider Enzyme, der Epimerase und der Lyase, aufeinander abgestimmt werden und z.B. die inhibierende Wirkung des Pyruvats auf die Epimerase durch mindere Pyruvatkonzentrationen zurückgedrängt werden. Das im Reaktionssystem vorhandene Pyruvat bildet selbst bei relativ geringen Konzentrationen einen erheblichen Überschuß gegenüber dem entstehenden ManNAc, so daß die Umsetzung zur Neu5Ac glatt verläuft unter Verbrauch von ManNAc, dessen Bildung aus GlcNAc dadurch begünstigt wird.

Geeignete Konzentrationen im Reaktor liegen bei 50 - 500 mMol GlcNAc/l insbesondere bei etwa 200 mMol GlcNAc/l und 30 - 200 mMol Pyruvat/l insbesondere 50 - 150 mMol Pyruvat/l, speziell um etwa 80 mMol Pyruvat/l.

Vorzugsweise wird kontinuierlich und insbesondere in einem Enzymmembranreaktor mit einer Ultrafiltrationsmembran mit einem cut-off von ≳ 1000 u gearbeitet.

Im Enzymmembranreaktor (EMR) werden die in löslicher Form darin vorliegenden Enzyme durch die vor dem Reaktorausgang befindliche Ultrafiltrationsmembran mit entsprechendem cut-off zurückgehalten. Eine Trägerfixierung mit entsprechenden Aktivitätsminderungen erübrigt sich daher. Der Reaktor kann vor Betriebsbeginn sterilisiert werden, so daß auf die Zugabe antibakteriell wirksamer Mittel verzichtet werden kann.

Da der EMR unter Auslaufbedingungen arbeitet, kann ebenfalls auf Puffersubstanzen verzichtet werden, wenn der Einlauf-pH entsprechend eingestellt wird. Der Verzicht auf diese Stoffe erleichtert die spätere Produktisolierung, die analog zu beschriebenen Verfahren erfolgt (vgl. R. Schauer a.a.o.).

Die beigefügten Zeichnungen dienen dem besseren Verständnis der Erfindung. Es zeigen im einzelnen:
- Figur 1, 2 und 4: Kurven für die relative Aktivität der Enzyme in Abhängigkeit vom pH-Wert;
- Figur 2A: die relative Aktivität der Epimerase in Abhängigkeit von der Pyruvat-Konzentration;
- Figur 3: die Abhängigkeit der Gleichgewichtskonstanten der Neu5Ac-Bldg. von der Temperatur;
- Figur 5: den Gleichgewichtsumsatz in Abhängigkeit vom Pyr/ManNAc-Verhältnis für unterschiedliche ManNAc-Konzentrationsbereiche; und
- Figur 6: das Elutionsverhalten von ManNAc, Neu5Ac und Pyr-H auf einer Anionenaustauschersäule.

Der Einsatz sowohl der Epimerase als auch der Lyase in demselben Reaktionssystem macht die Auswahl geeigneter Reaktionsbedingungen notwendig, die der enzymatischen Aktivität beider Enzyme angepaßt ist:

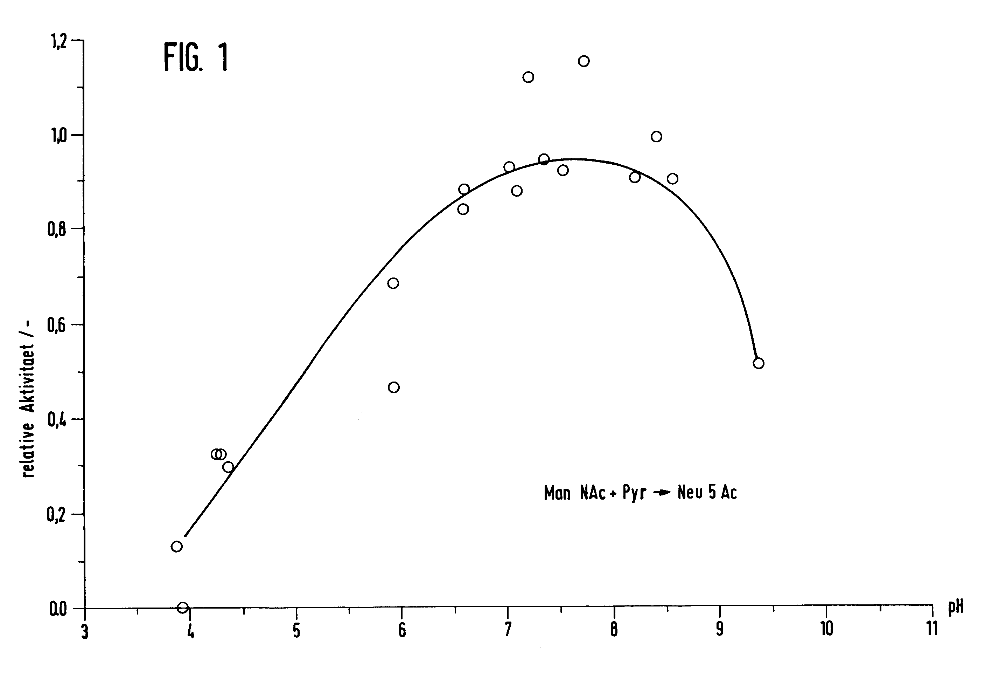

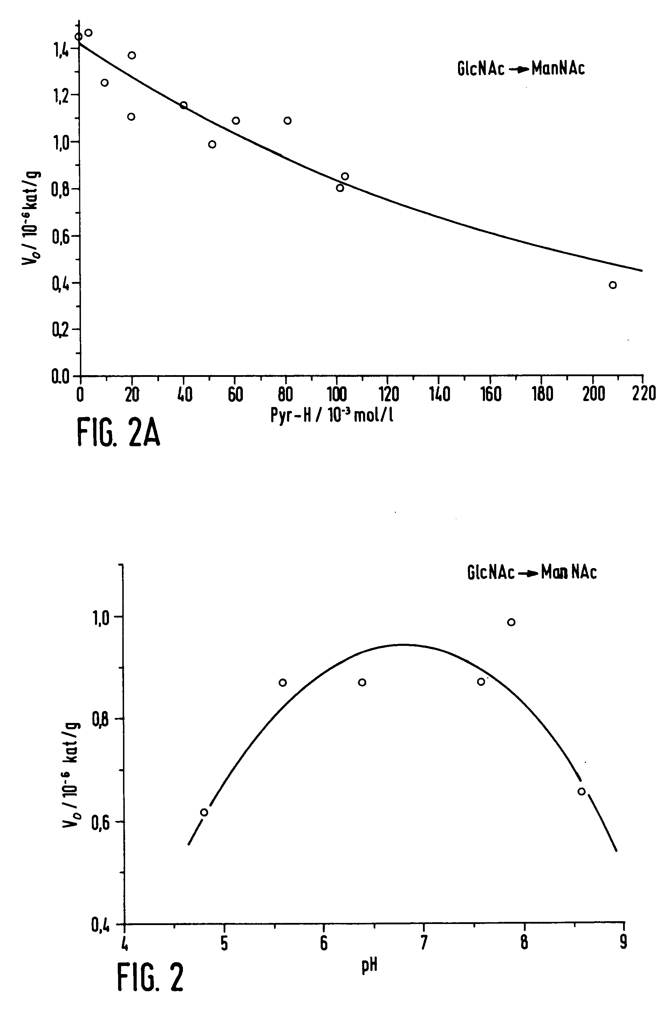

Es wurde daher zunächst die pH-Abhängigkeit der enzymatischen Reaktionen sowohl für die Neu5Ac-Bildung mitttels Lyase als auch der ManNAc-Bildung mittels Epimerase untersucht: Die angefügten Figuren 1 und 2 zeigen die für 25°C gefundenen Kurven, aus denen hervorgeht, daß ein um den Neutralbereich (pH 7) herum gewählter pH-Wert für beide Reaktionen günstig ist.

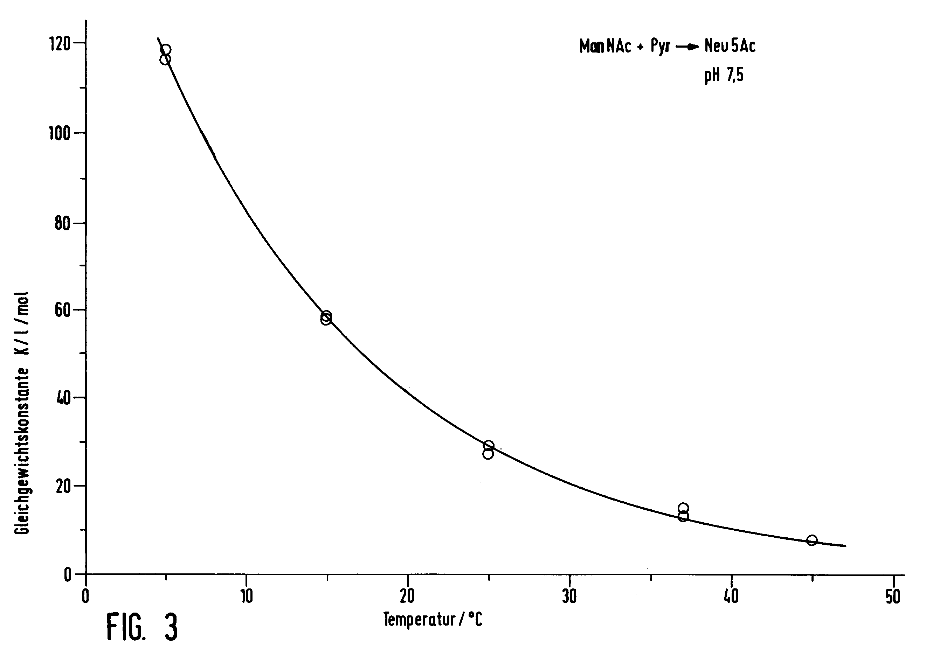

Die Untersuchung der Temperaturabhängigkeit der Gleichgewichtskonstanten der Neu5Ac-Bildung bei pH 7,5 (s. Figur 3) ergab, daß für die Neu5Ac-Bildung grundsätzlich möglichst niedrige Temperaturen nützlich sein sollten: Da jedoch bei niedriger Temperatur (von z.B. 10°C) eine deutliche Verminderung der Enzymaktivität auftritt und die für die Umsetzung als vorgelagerte Reaktionen geschwindigkeitsbestimmenden Mutarotationseffekte der Zucker ( α-Anomer 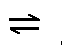 β-Anomer) bei niedrigen Temperaturen weniger rasch ablaufen, erscheinen Arbeiten um 25°C zweckmäßig.

Wie Figur 2A zeigt, wird die Isomerisierung von GlcNAc zu ManNAc durch Brenztraubensäure mit steigender Konzentration derselben zunehmend gehemmt. Weitere Versuche zeigen, daß auch die als Produkt gewünschte N-Acetylneuraminsäure inhibierend auf die Epimerase wirkt.

In den EMR werden für die im gleichen Reaktionssystem ablaufende zweistufige Synthese GlcNAc und Pyruvat eingespeist. Bei Anwesenheit der Epimerase und Lyase im Reaktor findet dann zunächst eine Umwandlung von GlcNAc in ManNAc mittels der Acylglucosamin-2-Epimerase (E.C. 5.1.3.8; Datta, Methods in Enzymology 41 (1975) 407-411), nach folgender Gleichung statt: 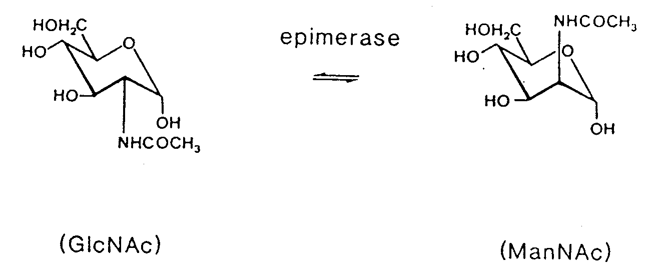
Das sich bildende ManNAc sieht sich dann einem großen Überschuß an Pyruvat gegenüber, wodurch die anschließende Umsetzung zur Neu5Ac gefördert wird.

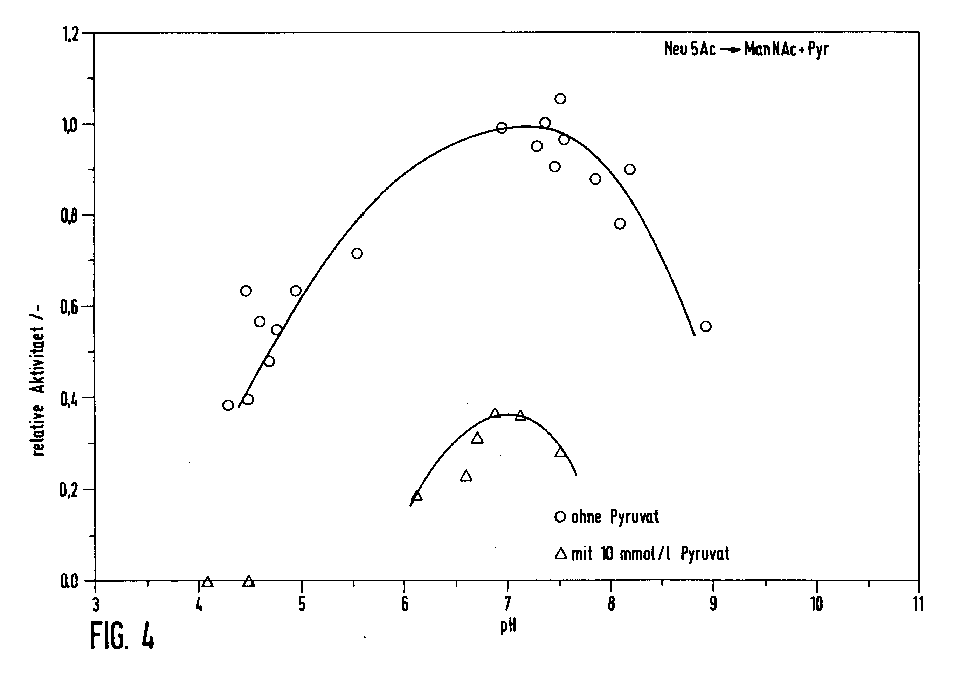

Deren Rückreaktion wird ferner durch die im Vergleich zur gebildeten Neu5Ac in erheblichem Überschuß vorhandene Pyruvatmenge stark gehemmt, wie aus Figur 4 zu entnehmen ist.

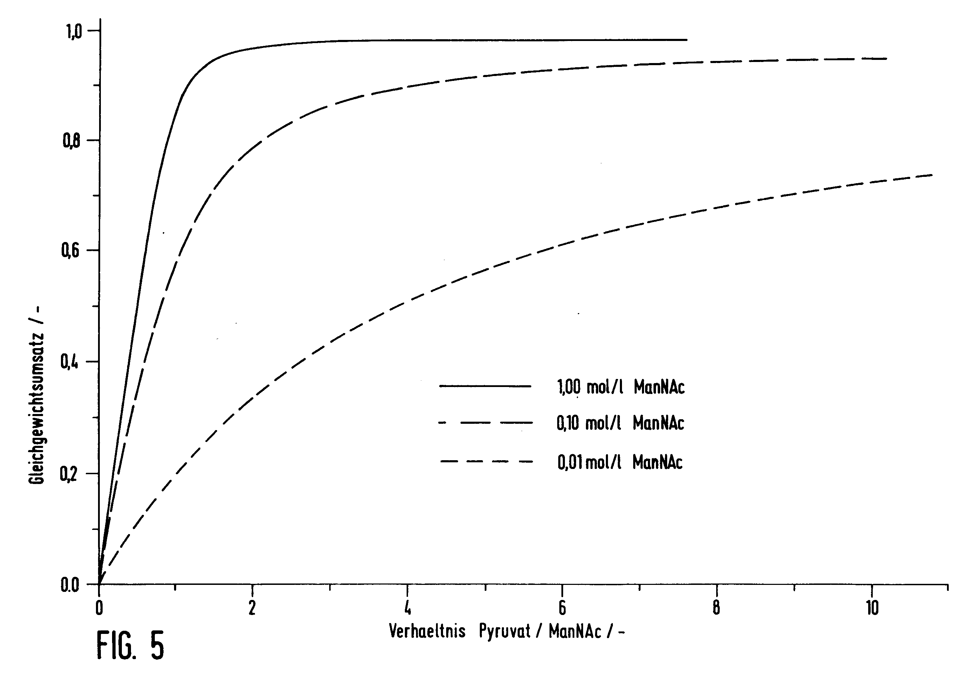

Figur 5 zeigt schließlich, daß durch die naturgemäß im erfindungsgemäßen Reaktionssystem im starken Überschuß vorhandene Pyruvatmenge die Verlagerung der Gleichgewichtskonstanten der Neu5Ac-Bildung zu höheren Werten begünstigt wird.

Da die Isomerisierung und Neu5Ac-Bildung im gleichen System mit Hilfe der gleichzeitig anwesenden Enzyme nebeneinander ablaufen sollen, ist es wichtig, daß die Aktivitäten der Enzyme aufeinander abgestimmt sind, wobei davon auszugehen ist, daß im Gleichgewicht etwa gleiche Umsätze erreicht werden sollten.

Danach erscheint ein Aktivitätsverhältnis beider Enzyme im Reaktor zueinander nützlich, dessen Wert dem Kehrwert des Quotienten aus den jeweiligen enzymatischen Umsetzungsgeschwindigkeiten entspricht.

Bei der Einspeisung der Ausgangsstoffe GlcNAc und Pyruvat ist zu berücksichtigen, daß der GlcNAc-Menge eine beherrschende Funktion zukommt, so daß dieses in zumindest vergleichbaren molaren Konzentrationen im Reaktor vorliegen sollte, vorzugsweise jedoch im Überschuß gegenüber Pyruvat angewandt wird. Große Pyruvatüberschüsse sollten vermieden werden, da die Epimerase durch Pyruvat inhibiert wird.

Die gebildete Neu5Ac unterliegt entsprechend der Gleichgewichtslage durch Rückreaktion einer mehr oder minder starken erneuten Aufspaltung. Diese Reaktion wird zwar durch Pyruvatüberschuß gehemmt (siehe Figur 4), jedoch erscheint es zweckmäßig, kontinuierlich Reaktionsmedium aus dem EMR über eine Ionenaustauschersäule zu schicken und den Rest in den Reaktor zurückzuführen. Zwei im Wechsel betriebene Säulen sind zweckmäßig.

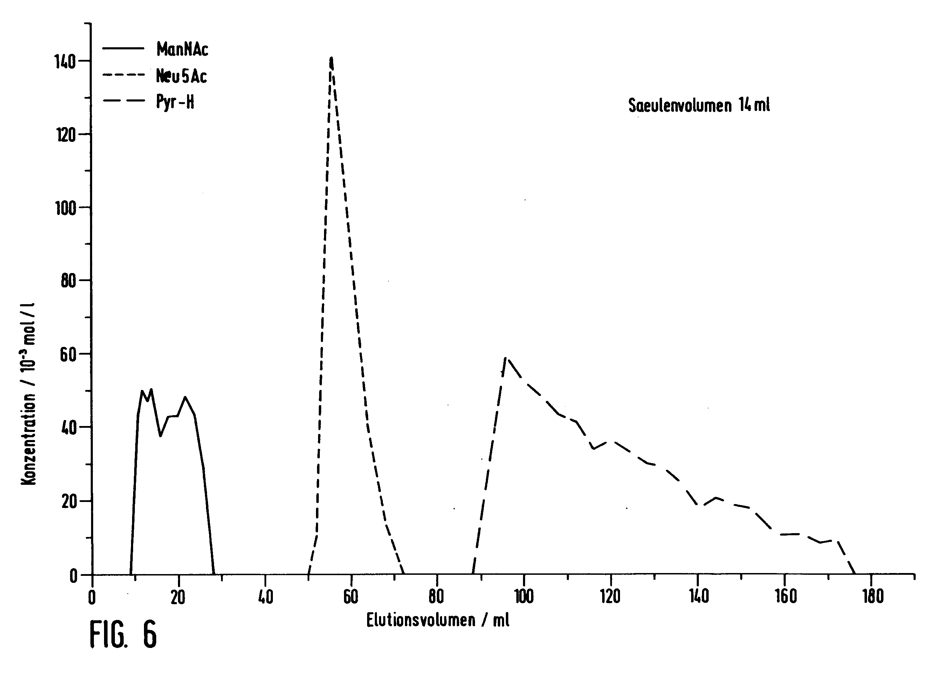

Wie Figur 6 zeigt, wird auf einer Anionenaustauschersäule (Dowex 1 x 2, Formiat, 25°C) Neu5Ac stärker als ManNAc festgehalten und kann somit auf der Säule angereichert werden. Ebenfalls gebundenes Pyr muß entsprechend ersetzt werden.

Für die optimierte Gesamtumsetzung erscheinen nach bisher vorliegenden Erfahrungen Verweilzeiten von 0,2 - 10 std., insbesondere von ca. 4 std., nützlich.

In Anbetracht des gefundenen breiten pH-Bereichs für abgestimmte Reaktionen, wie aus Figur 1 und 2 hervorgeht, kann ohne nennenswerten Aktivitätsverlust zwischen pH 6 und 8 gearbeitet werden. Dieser breite Bereich erleichtert das Arbeiten im kontinuierlichen Betrieb ohne Verwendung von Puffersubstanzen, da kleine pH-Schwankungen sich kaum auf den erzielten Umsatz auswirken. Der Verzicht auf Puffersubstanzen ist deshalb erstrebenswert, weil dadurch die Aufarbeitung erleichtert wird.

Puffersubstanzen sind normalerweise ionische Verbindungen, die auf dem zur Produktisolierung verwendeten Anionenaustauscher ebenfalls gebunden werden und durch geeignete chromatographische Bedingungen getrennt werden müßten. Ein typisches Chromatogramm ist in Figur 6 dargestellt. Es wurde ein Anionenaustauscher Dowex 1X2, Formiat-Form, verwendet. Als Elutionsmittel diente Ameisensäure, 1 mol/l. Ein scale up um den Faktor 8 wurde für die Säule durchgeführt, wobei die Trennleistung erhalten blieb.

### Beispiel für die enzymkatalysierte Produktion von N-Acetylneuraminsäure im Enzym-Membran-Reaktor

### Zulaufkonzentrationen der Substrate

| | |
|---|---|
| N-Acetylglucosamin | 200 · 10⁻³ mol/l |
| Na-Pyruvat | 100 · 10⁻³ mol/l |
| ATP | 5 · 10⁻³ mol/l |
| MgCl₂ · 6 H₂O | 5 · 10⁻³ mol/l |

Die Substrate werden in Wasser gelöst. Mit verdünnter Natronlauge wird ein pH von 7,2 eingestellt.

### Ezymkonzentrationen im Reaktor

| | |
|---|---|
| Epimerase | 11,9 mg/ml |
| Aldolase | 3,4 mg/ml |

### Betriebsbedingungen

| | |
|---|---|
| Temperatur | 25°C |
| pH 7,2 bis 7,5 am Reaktorauslauf | |
| Reaktorvolumen | 12 ml |
| Verwelzeit | 2,85 h |

### Ergebnis

Am Reaktorauslauf werden folgende Konzentrationen gemessen:

| | |
|---|---|
| - N-Acetylneuraminsäure | 35 mmol/l |
| - N-Acetylmannosamin | 20 mmol/l |

Bezüglich des eingesetzten Na-Pyr wird ein Umsatz von 35 % erreicht. Die Raum-Zeit-Ausbeute für N-Acetylneuraminsäure beträgt 109 g/(l · d).

## Patentansprüche

1. Verfahren zur Herstellung von N-Acetylneuraminsäure durch (a) Isomerisierung von N-Acetylglucosamin in Gegenwart von N-Acylglucosamin-2-Epimerase (E.C. 5.1.3.8) zu N-Acetylmannosamin, das dann (b) in Gegenwart von N-Acetylneuraminsäure-Pyruvat-Lyase (E.C. 4.1.3.3) mit Brenztraubensäure zu N-Acetylneuraminsäure umgesetzt wird,
**dadurch gekennzeichnet,**
daß man die N-Acetylneuraminsäurebildung in einem Reaktor herbeiführt, der sowohl die Epimerase als auch die Lyase enthält und in den N-Acetylglucosamin und Pyruvat eingespeist werden und aus dessen Ablauf die N-Acetylneuraminsäure gewonnen wird.

2. Verfahren nach Anspruch 1,
**dadurch gekennzeichnet,**
daß man die Umsetzung kontinuierlich durchführt.

3. Verfahren nach Anspruch 2,
**dadurch gekennzeichnet,**
daß man die Umsetzung in einem Enzymmembranreaktor mit einer Ultrafiltrationsmembran mit einem cut-off vom ≳ 1000 u durchführt.

4. Verfahren nach einem der vorangehenden Ansprüche,
**dadurch gekennzeichnet,**
daß die Epimerase und die Lyase im Reaktor in einem Aktivitätsverhältnis vorliegen, das dem Kehrwert des Quotienten aus den jeweiligen enzymatischen Umsetzungsgeschwindigkeiten entspricht.

5. Verfahren nach einem der vorangehenden Ansprüche,
**dadurch gekennzeichnet,**
daß im Reaktor für einen Überschuß an N-Acetylglucosamin gegenüber Pyruvat gesorgt wird, das gegebenenfalls nachdosiert wird.

6. Verfahren nach einem der vorangehenden Ansprüche,
**dadurch gekennzeichnet,**
daß man den Reaktorablauf durch eine Ionenaustauschersäule zur Abtrennung der N-Acetylneuraminsäure schickt und den Rest in den Reaktor zurückführt.

7. Verfahren nach einem der vorangehenden Ansprüche,
**dadurch gekennzeichnet,**
daß man die enzymatischen Reaktionen bei pH 7,5 und 25°C ablaufen läßt.

8. Verfahren nach einem der Ansprüche 3 bis 7,
**dadurch gekennzeichnet,**
daß mit mittleren Verweilzeiten von 0,2 - 10 h, insbesondere von 4 h gearbeitet wird.

## Claims

1. A process for producing N-acetylneuraminic acid by (a) the isomerisation of N-acetylglucosamine in the presence of N-acetylglucosamine-2-epimerase (E.C. 5.1.3.8) to form N-acetylmannosamine, which is (b) then reacted with pyruvic acid in the presence of N-acetylneuraminic acid-pyruvate-lyase (E.C. 4.1.3.3) to form N-acetylneuraminic acid,
characterised in that the formation of N-acetylneuraminic acid is conducted in a reactor which contains both the epimerase and the lyase and into which N-acetylglucosamine and pyruvate are fed, and from the discharge of which the N-acetylneuraminic acid is obtained.

2. A process according to claim 1, characterised in that the reaction is conducted continuously.

3. A process according to claim 2, characterised in that the reaction is conducted in an enzyme membrane reactor having an ultrafiltration membrane with a cut-off of ≳ 1000 u.

4. A process according to any one of the preceding claims, characterised in that the epimerase and the lyase are present in the reactor in an activity ratio which corresponds to the reciprocal of the ratio of the respective enzymatic reaction rates.

5. A process according to any one of the preceding claims characterised in that an excess of N-acetylglucosamine over pyruvate, which is optionally metered in secondarily, is ensured in the reactor.

6. A process according to any one of the preceding claims, characterised in that the reactor discharge is passed through an ion-exchange column to separate out the N-acetylneuraminic acid and the residue is recycled to the reactor.

7. A process according to any one of the preceding claims, characterised in that the enzymatic reaction is caused to proceed at pH 7.5 and 25°C.

8. A process according to any one of claims 3 to 7, characterised in that average residence times of 0,2 - 10 hours, particularly of 4 hours, are employed.

## Revendications

1. Procédé de préparation d'acide N-acétylneuraminique par (a) isomérisation de N-acétylglucosamine en présence de N-acylglucosamine-2-épimérase (E.C. 5.1.3.8) en N-acétylmannosamine qu'on fait réagir ensuite (b) en présence d'acide N-acétylneuraminique-pyruvate-lyase (E.C. 4.1.3.3) avec de l'acide pyruvique pour former de l'acide N-acétylneuraminique, caractérisé en ce qu'on effectue la formation de l'acide N-acétylneuraminique dans un réacteur qui contient l'épimérase ainsi que la lyase et dans lequel on introduit de la N-acétylglucosamine et du pyruvate et dont l'issue on obtient l'acide N-acétylneuraminique.

2. Procédé selon la revendication 1, caractérisé en ce qu'on effectue la réaction en continu.

3. Procédé selon la revendication 2, caractérisé en ce qu'on effectue la réaction dans un réacteur enzymatique à membrane avec une membrane d'ultrafiltration ayant une limite d'exclusion ≳ 1000 u.

4. Procédé selon l'une des revendications précédentes, caractérisé en ce que l'épimérase et la lyase sont présentes dans le reacteur à un rapport d'activité qui correspond à la valeur inverse du quotient des vitesses respectives de réaction enzymatique.

5. Procédé selon l'une des revendications précédentes, caractérisé en ce qu'on veille à ce que le réacteur présente un excès de N-acétylglucosamine par rapport au pyruvate, qu'on renouvelle éventuellement.

6. Procédé selon l'une des revendications précédentes, caractérisé en ce qu'on amène le produit d'écoulement du réacteur dans une colonne échangeuse d'ions pour séparer l'acide N-acétylneuraminique et on recycle le reste dans le réacteur.

7. Procédé selon l'une des revendications précédentes, caractérisé en ce qu'on laisse les réactions enzymatiques se dérouler à pH 7,5 et à 25°C.

8. Procédé selon l'une des revendications 3 à 7, caractérisé en ce qu'on travaille avec des temps moyens de séjour de 0,2 à 10 heures, en particulier de 4 h.
